# EUROPEAN PATENT SPECIFICATION

(11) **EP 4 559 507 B1**
(45) Date of publication and mention of the grant of the patent: **03.06.2026**
(21) Application number: 23211521.2
(22) Date of filing: 22.11.2023
(51) Int. Cl.: A61N 1/05, A61N 1/362

(54) **IMPLANTABLE MEDICAL DEVICE FOR A COMBINED CARDIAC RESYNCHRONIZATION THERAPY AND CARDIAC CONTRACTILITY MODULATION THERAPY**
IMPLANTIERBARE MEDIZINISCHE VORRICHTUNG FÜR EINE KOMBINIERTE KARDIALE RESYNCHRONISATIONSTHERAPIE UND KARDIALE KONTRAKTILITÄTSMODULIERUNGSTHERAPIE
DISPOSITIF MÉDICAL IMPLANTABLE POUR UNE THÉRAPIE DE RESYNCHRONISATION CARDIAQUE ET UNE THÉRAPIE DE MODULATION DE CONTRACTILITÉ CARDIAQUE COMBINÉES

(43) Date of publication of application: 28.05.2025
(73) Proprietor: BIOTRONIK SE & Co. KG, 12359 Berlin (DE)
(72) Inventor: Dörr, Thomas, 12437 Berlin (DE)
(74) Representative: Biotronik Corporate Services SE

(56) References cited:
- US-A1- 2003 199 956
- US-A1- 2013 006 318
- US-A1- 2023 001 204

## Description

The present invention relates to an implantable medical device according to the preamble of claim 1.

Implantable medical devices for stimulating a human or animal heart can feature different functionalities. To give an example, a CRT-D device is designed and arranged to accomplish a cardiac resynchronization therapy and a defibrillation of the patient's heart. Such a CRT-D device typically has three electrodes, namely a combined right ventricular defibrillation and stimulation electrode, a right atrial stimulation and sensing electrode and a left ventricular coronary sinus electrode. Some manufacturers like Biotronik also offer a more complex right ventricular electrode that integrates the atrial sensing functionality into the right ventricular stimulation electrode. However, still in this case, two electrodes are necessary to be implanted in or at the patient's heart. Thus, the solutions known from prior art require for a biventricular stimulation at least two ventricular electrodes. This requires a very big connecting block (header) of the stimulation generator of the implantable medical device being able to receive at least two, but typically even three electrode connectors. Such a big connecting block complicates the implantation of the implantable medical device.

If the implantable medical device is designed and arranged as a CRT-P device, i.e., a device for cardiac resynchronization therapy and pacing (but no defibrillation), the general setup is almost identical to the previously described CRT-D device. However, the CRT-P device does not comprise a defibrillation electrode. Nonetheless, CRT-P devices known from prior art typically require three distinct electrodes and an accordingly big connector block of the stimulation generator to connect these electrodes to the stimulation generator.

If the implantable medical device is designed and arranged as a device for employing a two-chamber therapy, it is also necessary to implant two distinct electrodes into the patient's heart. One electrode is guided into the right atrium, and the other is guided into the left ventricle. Both electrodes need to be connected with the stimulation generator, i.e., the implantable pulse generator. For this purpose, the implantable pulse generator typically comprises at least two connecting sockets. Like in case of a three-socket connecting box, this requires a significant amount of space.

As outlined above, prior art already teaches a specific variant of integrated electrodes that uses a proximal bipole for sensing electric signals in the patient's right atrium. Then, this variant of the ventricular electrode already takes over the functionality of the atrial electrode. However, this electrode comprises a switch with two plugs to be able to be connected with a regular two-chamber stimulation system. Thus, the integration of the atrial electrode into the ventricular electrode does not alter the space requirement of the connector box of the stimulation generator.

A cardiac contractility modulation (CCM) device is used for delivering a non-stimulatory energy to a patient's heart. This non-stimulatory energy increases the cardiac (in particular ventricular) contractility that, in turn, helps for increasing cardiac output. In CCM therapy, electrical stimulation pulses are typically delivered to the heart during a refractory period of the heart, such as a time immediately following a cardiac contraction. During such a refractory period, the cardiac tissue is insensitive to electrical stimulation pulses; the delivered pulses will not result in a cardiac contraction. However, the delivered CCM stimulation pulses are capable of increasing heart contractility. As a result, the following cardiac contraction can be more powerful, leading to a higher cardiac output.

A cardiac cycle comprises two refractory periods, an absolute refractory period and a relative refractory period. The absolute ventricular refractory period begins at the start of the action potential leading to ventricular contraction. It includes the so-called the QRS complex and the rising portion of the so-called T wave following the QRS complex. The relative refractory period includes the falling portion of the T wave. During the absolute ventricular refractory period, ventricular tissue cannot be stimulated to contraction. During the relative ventricular refractory period, ventricular tissue can be generally stimulated to contraction. However, a larger stimulus than normal is typically required. CCM stimulation pulses are typically delivered during the absolute ventricular refractory period.

If the sensing and detecting functionalities of an implantable medical device are integrated into one or two electrodes, there remains the requirements of providing a patient with an optimum stimulation adapted to the patient's health status.

WO 2010/003190 A2 describes an implantable cardiac rhythm/function management system that integrates cardiac contractility modulation (CCM) and one or more other therapies, such as pacing, defibrillation/cardioversion, cardiac resynchronization therapy (CRT), or neurostimulation.

WO 2022/208339 A1 describes a method of cardiac signal processing including: receiving measurements from at least two electrodes positioned within the heart; determining, using the measurements, relative positioning of the at least two electrodes relative to each other; evaluating suitability of the relative positioning of the at least two electrodes for measurement of cardiac activity to determine which cardiac cycles should receive cardiac contractility modulation stimulation. Document US-A-2003/199956 discloses an implantable cardiac stimulator.

It is an object of the present invention to provide an implantable medical device that has a small space requirement, allows an easy implantation, and enables a comprehensive cardiac resynchronization therapy and a cardiac contractility modulation therapy.

This object is achieved with an implantable medical device for stimulating a human or animal heart having the features of claim 1.

Such an implantable medical device comprises a processor, a memory unit, a stimulation unit, and a detection unit. The stimulation unit is arranged and designed to stimulate a human or animal heart. The detection unit is designed and arranged to detect an electric signal of the same heart. In addition, the implantable medical device comprises at least one electrode forming part of the stimulation unit and of the detection unit.

What is special about the presently claimed and described implantable medical device is that the at least one electrode comprises a distal bipole and is configured to be implanted within the septum of the heart to be stimulated. At this implantation site, the distal bipole of the at least one electrode is able to stimulate at least the left ventricle of the patient's heart by left bundle branch area pacing (LBBAP). Thus, the at least one electrode is able to bypass a left bundle branch block and thus to achieve an efficient pacing of the left ventricle even in case that the physiologic stimulus lines are no longer working or no longer working correctly.

The memory unit of the implantable medical device comprises a computer-readable program that causes the processor to perform the step explained in the following when being executed on the processor.

This step comprises delivering a left bundle branch atrial pacing (LBBAP) stimulation and a cardiac contractility modulation (CCM) stimulation to the heart to be stimulated. In this context, the cardiac contractility modulation stimulation is delivered temporally offset to the left bundle branch area pacing stimulation. In doing so, the optimum time point for delivering the LBBAP stimulation and the CCM stimulation can be chosen. This results in a better efficacy of the delivered stimulation pulses and thus a higher efficacy of the therapy provided or delivered by the implantable medical device.

As explained above, the cardiac contractility modulation stimulation will not stimulate the heart in a classic sense, i.e., it will not lead to contraction of the ventricle of the heart. Rather, the cardiac contractility modulation stimulation serves for increasing the contractility of the heart, leading to an increased cardiac output. While the left bundle branch area pacing stimulation serves for keeping the normal cardiac activity and thus for regular, synchronized contractions of the ventricles to pump blood through the patient's body, the cardiac contractility modulation stimulation serves for ameliorating cardiac parameters and lastly the cardiac output. A combination of left bundle branch area pacing stimulation and cardiac contractility modulation stimulation thus ameliorates the cardiac state of the patient to whom the combined therapy is applied. The presently claimed and described implantable medical device is a particularly cost-saving system comprising only a minimum number of electrodes to be implanted for applying such a combined cardiac contractility modulation stimulation and left bundle branch area pacing stimulation (aiming at a cardiac resynchronization of the patient's heart).

In an embodiment, the at least one electrode comprises at its distal terminus a helix. This helix is designed and configured to be secured within cardiac tissue. For this purpose, the helix can be turned into the cardiac tissue, i.e., into the septum of the patient's heart. After having implanted the at least one electrode into the septum, in particular into the deep septum, it is possible to achieve an effective stimulation of the left ventricle even if no electrode is directly placed within the left ventricle or on an outside thereof (as in case of prior art left ventricular stimulation electrodes). An implantation of the distal bipole of the at least one electrode in deep septum at a position distally of the left branch block enables left bundle branch area pacing without requiring a separate left ventricular electrode.

In an embodiment, the helix is designed as fixed fixing helix. In another embodiment, the helix is designed as unscrewable fixing helix. Either design is particularly appropriate for fixing the at least one electrode within the septum of the patient's heart.

In an embodiment, the helix forms at least a part of the distal bipole of the at least one electrode. Expressed in other words, at least one electrode pole (in particular both electrode poles) of the distal bipole of the at least one electrode is realized by the helix that is also used to secure the at least one electrode within the septum of the patient's heart. This guarantees a very efficient energy transfer from the at least one electrode into the surrounding cardiac tissue.

In an embodiment, the delivered cardiac contractility modulation stimulation comprises 1 to 10 pulses, in particular 2 to 9 pulses, in particular 3 to 8 pulses, in particular 4 to 7 pulses, in particular 5 to 6 pulses.

In an embodiment, each pulse provided for the cardiac contractility modulation stimulation has two phases with opposite polarities and predeterminable duration.

In an embodiment, the phase duration of each phase lies in a range of from 1 to 10 ms, in particular from 2 to 8 ms, in particular from 3 to 7 ms, in particular from 4 to 6.5 ms, in particular from 4.5 to 6.0 ms, in particular from 5.0 to 5.5 ms. In an embodiment, the duration of both phases of an individual pulse is equally long.

In an embodiment, the amplitude of a pulse of the cardiac contractility modulation stimulation lies in a range of from 2.0 V to 10.0 V, in particular from 2.5 V to 9.5 V, in particular from 3.0 V to 9.0 V, in particular from 3.5 V to 8.5 V, in particular from 4.0 V to 8.0 V, in particular from 4.5 V to 7.5 V, in particular from 5.0 V to 7.0 V, in particular from 5.5 V to 6.5V, in particular from 5.0 V to 6.0 V.

The distal bipole comprises a first electrode pole and a second electrode pole located proximally from the first electrode pole. In an embodiment, a distance between a distal end of the second electrode pole and a proximal end of the first electrode pole lies in a range of from 1 mm to 30 mm, in particular from 2 mm to 25 mm, in particular from 3 mm to 20 mm, in particular from 4 mm to 15 mm, in particular from 5 mm to 10 mm. Such a distance between the first electrode pole and the second electrode pole is particularly appropriate to allow a stimulation of different cardiac regions by the first electrode pole and the second electrode pole after the electrode has been implanted into the septum of the patient's heart. Then, the first electrode pole can stimulate the left bundle branch, i.e., it can perform left bundle branch area pacing (LBBAP). Likewise, the second electrode pole can then stimulate the right bundle branch, i.e., it can perform right bundle branch area pacing (RBBAP). In addition, the second electrode pole can particularly well detect right ventricular signals in this position.

In an embodiment, the at least one electrode comprises - besides the distal bipole - a proximal bipole. This proximal bipole is arranged and designed to detect an intrinsic atrial signal of the heart to be stimulated. After implantation of the at least one electrode of the implantable medical device, the distal bipole is implanted within the septum of the heart to be stimulated, wherein the proximal bipole is located within the right atrium of the heart to be stimulated. The intrinsic atrial signal sensed by the proximal bipole can then be used to trigger the further stimulation pulses in order to deliver the LBBAP stimulation and the CCM stimulation to the ventricle of the heart to be stimulated.

In an embodiment, the at least one electrode comprises - besides the distal bipole and besides the optional proximal bipole - a shock coil. This shock coil is arranged proximally from the distal bipole. Such a shock coil is particularly appropriate to provide a defibrillation shock to the heart to be stimulated. Then, the implantable medical device can be used as CRT-D device.

In an embodiment, the shock coil has a surface of at least 150 mm², in particular at least 175 mm², in particular at least 200 mm², in particular at least 225 mm², in particular at least 250 mm². Such a surface enables a sufficiently big shock pulse to be delivered by the shock coil to achieve an efficient cardiac defibrillation of the patient's heart.

In an embodiment, the implantable medical device comprises a second electrode that forms at least part of the stimulation unit. The second electrode is configured to be implanted within the septum of the heart to be stimulated. Typically, the site of implantation of the second electrode is different from the site of implantation of the at least one electrode referred to above. While the at least one electrode referred to above is implanted distally of a left bundle branch block, the second electrode is to be implanted in a more proximal position. Since the second electrode is intended to be used for delivering the cardiac contractility modulation stimulation, it is not necessary that the stimulation pulses delivered by the second electrode would indeed be able to effect a contraction of the ventricle of the heart to be stimulated (such stimulation is not intended by the cardiac contractility modulation stimulation). The second electrode can be used for splitting up the LBBAP and the CCM functionalities of the implantable medical device so that one of the two electrodes (in particular the at least one electrode referred to above) is used for delivering the LBBAP stimulation, whereas the other electrode (in particular the second electrode) is used for delivering the CCM stimulation.

In an embodiment, the implantable medical device comprises exactly two electrodes and no additional electrodes.

As explained above, the at least one electrode of the implantable medical device may be equipped with a shock coil for delivering a defibrillation pulse to the heart to be stimulated. In an embodiment, the shock coil is not integrated into the at least one electrode comprising the distal bipole but is rather present on a separate defibrillation electrode. This separate defibrillation electrode forms part of the stimulation unit. It is configured to be implanted within the right ventricle of the heart to be stimulated. In this context, the separate defibrillation electrode is typically not implanted into the septum of the heart to be stimulated but is rather loosely placed into the right ventricle of the heart to be stimulated or is anchored into the apex in the area of the right ventricle of the heart to be stimulated.

Considering the implantation effort of the implantable medical device, one would typically choose an embodiment in which the defibrillation functionality is integrated into the at least one electrode having a distal bipole. Then, only this electrode needs to be implanted in a minimum version of the implantable medical device. However, in some instances it may be desired to separate the defibrillation functionality from the LBBAP stimulation functionality of the implantable medical device. This may be the case, e.g., for keeping the complexity of the at least one electrode low.

To provide a highly integrated implantable medical device that can feature all of its functionalities with very low space requirement and that allows a particularly simple implantation, the implantable medical device comprises, in an embodiment, only a single electrode. Then, only this single electrode needs to be implanted into the heart to be stimulated. Prior art systems featuring a cardiac resynchronization therapy and a cardiac contractility modulation therapy typically require two housings and at least four electrodes that all need to be implanted into or adjacent to the heart to be stimulated. This makes the whole implantation procedure significantly more difficult than in case of the presently claimed and described implantable medical device.

In an embodiment, in particular in an embodiment that is combined with the precedingly explained embodiment featuring only a single electrode of the implantable medical device, the implantable medical device comprises a housing with a header that has only a single electrode connector receiving socket. This reduces the space requirement of the housing and facilitates implantation of the housing and thus of the overall implantable medical device with respect to prior art devices. If only a single electrode connector receiving socket is present, an electrode connector cannot be inadvertently inserted into a wrong electrode connector receiving socket. Thus, the implantation of the implantable medical device is less error-prone than the implantation of prior art devices.

Since the cardiac contractility modulation stimulation requires significant more electrical energy than a typical stimulation applied for anti-bradycardic pacing, anti-tachycardic pacing and/or cardiac resynchronization, the implantable medical device comprises, in an embodiment, a rechargeable battery. To allow for easy charging of the battery, the implantable medical device further comprises, in this embodiment, a device for charging the rechargeable battery by an external charging device. Such charging procedure can be accomplished, e.g., in a wireless manner by placing the external device closely to the implantable medical device and by allowing a charging of the rechargeable battery via induction. Due to the possibility of enabling a charging of the rechargeable battery, the lifetime of the implantable medical device is significantly increased. Without such charging functionality, the lifetime of the implantable medical device would be rather short as long as no batteries, which are suited to be integrated into the implantable medical device, having a higher capacity than the currently available batteries are available on the market.

In an embodiment, the computer-readable program causes the processor to detect a cardiac cycle of the heart to be stimulated. This detection is done with the detection unit of the implantable medical device. Additionally, the computer-readable program causes the processor to determine a refractory period of the cardiac cycle. Finally, the computer-readable program causes the processor to deliver the cardiac contractility modulation stimulation within the refractory period of the cardiac cycle. In doing so, it is ensured that the stimulation pulses delivered during the cardiac contractility modulation stimulation will not lead to a cardiac contraction, but will rather improve the contractility of the heart and thus achieve higher cardiac output in at least one, but typically a plurality of subsequent cardiac cycles.

In an embodiment, the refractory period is an absolute refractory period. In an embodiment, the refractory period is an (absolute) atrial and/or an (absolute) ventricular refractory period. Delivering the cardiac contractility modulation stimulation within the absolute right ventricular refractory period and in particular within the absolute right ventricular refractory period and the absolute right atrial refractory period is particularly appropriate.

In an embodiment, the implantable medical device comprises a communication unit. This communication unit serves for receiving instructions from an external device, wherein the instructions relate, e.g., to the activation and/or deactivation of individual functionalities of the implantable medical device. Then, it is possible to transmit according instructions (control signals) from a programming device or via remote programming to the implantable medical device. This enables a user-specific programming of the implantable medical device and thus enhances the versatility of the implantable medical device.

In an embodiment, the communication unit serves for transferring data to the processor in a wireless manner. All standard data transmission protocols or specifications are appropriate for such a wireless data communication. Examples of standard data transmission protocols or specifications are the Medical Device Radiocommunications Service (MICS), the Bluetooth Low Energy (BLE) protocol, the Zigbee specification, the long range wide area network (LoRaWAN) protocol, the wireless personal area network (WPAN) specification, the low-power wide-area network (LPWAN) specification, the wireless local area network (WLAN) specification, the Global System for Mobile Communications (GSM) specification, the Long-Term Evolution (LTE) standard, and the fifth-generation technology standard for broadband cellular networks (5G).

In an aspect, the present disclosure relates to a method for operating an implantable medical device according to the preceding explanations. This method comprises the steps explained in the following.

According to this method step, a stimulation trigger signal for a subsequent left bundle branch area pacing (LBBAP) stimulation and a stimulation trigger signal for a subsequent cardiac contractility modulation (CCM) stimulation of the heart to be stimulated are provided. In this context, the stimulation trigger signal for the CCM stimulation is provided temporally offset to the stimulation trigger signal for the LBBAP stimulation.

The provided stimulation trigger signal is - in a time frame lying outside the claimed range of the method of operating the implantable medical device - guided through the electrode to the distal bipole and can then effect a ventricular stimulation of the heart to be stimulated. In this context, the LBBAP stimulation is typically used to effect a cardiac resynchronization, whereas the CCM stimulation does not lead to a cardiac contraction, but rather to an enhanced cardiac output, as explained above.

In an embodiment, the method comprises some additional steps that will be explained in the following.

According to one of the additional steps, the detection unit of the implantable medical device is used for detecting a cardiac cycle of the heart to be stimulated.

According to a second of these further steps, a refractory period of the cardiac cycle is determined.

According to a third of these additional steps, the stimulation trigger signal for the cardiac contractility modulation stimulation is provided within the refractory period of the cardiac cycle. In doing so, it is ensured that the stimulation trigger signal will induce a subsequent cardiac contractility modulation stimulation within a timeframe of the cardiac cycle in which no further cardiac contraction can be achieved, but which will rather result in an ameliorated cardiac contractility and thus an enhanced cardiac output in a subsequent cardiac cycle.

In an aspect, the present disclosure relates to a medical method for providing a cardiac combination therapy, namely a combination of a cardiac resynchronization therapy and a cardiac contractility modulation therapy to a patient in need thereof. This method comprises the step explained in the following.

According to this method step, a stimulation unit of an implantable medical device for stimulating a human or animal heart is used for delivering a left bundle branch area pacing stimulation and a cardiac contractility modulation stimulation to a heart to be stimulated. These two different types of stimulation can be particularly well applied with an implantable medical device according to the above explanations. In this context, the cardiac contractility modulation stimulation is delivered temporally offset to the left bundle branch area pacing stimulation. In particular, the cardiac contractility modulation stimulation is provided slightly after the left bundle branch area pacing stimulation, e.g., within the same cardiac cycle as the left bundle branch area pacing stimulation.

In an embodiment, the method further comprises some additional steps that will be explained in the following.

According to a first of these additional steps, a detection unit of the implantable medical device is used for detecting a cardiac cycle of the heart to be stimulated.

According to a second of these additional method steps, a refractory period of the cardiac cycle is determined.

According to a third of these additional method steps, the cardiac contractility modulation stimulation is delivered within the refractory period of the cardiac cycle.

All embodiments of the implantable medical device can be combined in any desired way and can be transferred either individually or in any arbitrary combination to each of the methods. Likewise, all embodiments of each of the methods can be combined in any desired way and can be transferred either individually or in any arbitrary combination to the implantable medical device and to the respective other method.

Further details of aspects of the present invention will be explained in the following making reference to exemplary embodiments and accompanying Figures. In the Figures:
- Figure 1: shows a prior art system having CRT-D and CCM functionality implanted into a human heart;
- Figure 2: shows a first embodiment of an implantable medical device for combined LBBAP stimulation and CCM stimulation implanted into a human heart;
- Figure 3: shows a second embodiment of an implantable medical device for combined LBBAP stimulation and CCM stimulation implanted into a human heart; and
- Figure 4: schematically shows different components of an embodiment of an implantable medical device.

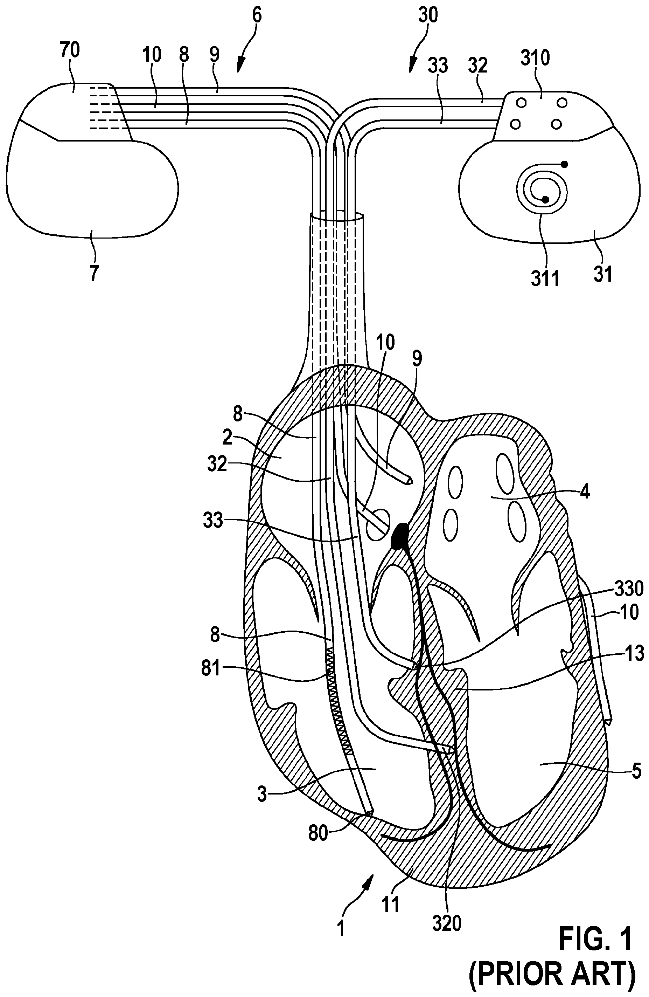

Figure 1 shows a human heart 1 comprising a right atrium 2, a right ventricle 3, a left atrium 4, and a left ventricle 5. A prior art device for cardiac resynchronization therapy and defibrillation (CRT-D device) 6 is implanted into the heart 1. This CRT-D device 6 comprises a stimulation generator 7 having a header 70. The header 70 comprises three header ports, each of which serves for receiving an individual electrode. Thus, the CRT-D device 6 comprises three electrodes, namely a right ventricular stimulation and shock electrode 8, a right atrial stimulation and sensing electrode 9, and a left ventricular stimulation and sensing electrode 10.

The right ventricular stimulation and shock electrode 8 is implanted into the right ventricle 3, wherein a tip 80 of the right ventricular stimulation and shock electrode 8 is placed close to the apex 11 or is implanted into the apex 11 of the patient's heart 1. The right ventricular stimulation and shock electrode 8 furthermore comprises a shock coil 81 that is located proximally of the tip 80 of the right ventricular stimulation and shock electrode 8 and is situated, in the implanted state of the right ventricular stimulation and shock electrode 8, within the right ventricle 3.

The right atrial stimulation and sensing electrode 9 is implanted into the right atrium 2 and is secured within the cardiac tissue surrounding the right atrium 2. The left ventricular stimulation and sensing electrode 10 is guided into a coronary vein and placed outside the left ventricle 5 to be able to stimulate the left ventricle 5.

It is apparent from Figure 1 that the implantation procedure of the CRT-D device 6 is rather complicated. In addition, the header 70 of the stimulation generator 7 requires a significant amount of space to be able to house the three electrodes 8, 9, 10.

To also enable a cardiac contractility modulation stimulation, the prior art system comprises a device for cardiac contractility modulation (CCM device) 30 that is also implanted into the heart 1. The CCM device 30 comprises a CCM stimulator 31 having a header 310 and a charging coil 311 that serves for charging a rechargeable battery contained within the CCM stimulator 31. The CCM device 30 furthermore comprises a first CCM electrode 32 and a second CCM electrode 33. Both the first CCM electrode 32 and the second CCM electrode 33 are implanted into the septum 13 of the heart 1. The actual site of implantation of the first CCM electrode 32 and the second CCM electrode 33 within the septum 13 is not of specific importance and can deviate from the schematic depiction of Figure 1. Typically, a CCM stimulation is achieved by applying a stimulation pulse between a distal electrode pole 320 of the first CCM electrode 32 and a distal electrode pole 330 of the second CCM electrode 33.

Summarizing, the prior art system for applying a combined CRT and CCM therapy requires altogether five electrodes to be implanted into the heart 1 as well as two housings for the respective stimulators. This makes an implantation procedure extremely difficult. In addition, the high number of electronic components implanted into the heart 1 presents a significant risk that at least one of these components might needed to be replaced due to a failure. Such replacement procedure is also extremely difficult in view of the limited space available within and around the heart 1.

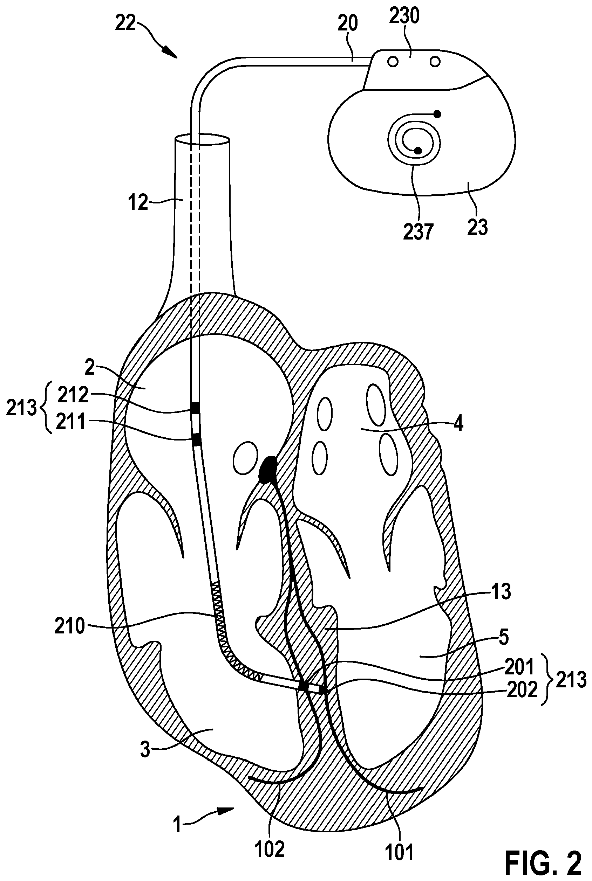

Figure 2 shows an embodiment of a combined CCM and CRT device 22 that is able to provide the same functionalities as the prior art system shown in Figure 1. However, the combined CCM and CRT device 22 comprises only a single electrode 20 that serves for sensing cardiac signals of the heart 1 and for stimulating the heart 1. In this and all following figures, similar elements will be denoted with the same numeral reference.

The combined CCM and CRT device 22 comprises a stimulator 23 having a header 230 and a charging coil 237. The header 230 is significantly smaller than the header 310 of the prior art stimulator 31 (confer Figure 1) since it comprises only a single electrode connector receiving socket to receive a connector of the single electrode 20. Due to the smaller dimensions of the stimulator 23 with its header 230, an implantation of the stimulator 23 is significantly easier than an implantation of the prior art stimulator 31 (confer Figure 1).

The electrode 20 is guided through the upper vena cava 12 into the heart 1. Here it is furthermore guided through the right atrium 2 and the right ventricle 3 into the septum 13 of the heart 1. The electrode 20 is secured with its distal tip within the septum 13 of the heart 1. The distal tip furthermore comprises a distal bipole 203 having a first distal electrode pole 201 and a second distal electrode pole 202. The electrode 20 (which can also referred to as biventricular electrode) is implanted in a deep septal position so that it can stimulate the left bundle branch 101 of the heart 1 and thus stimulate the left ventricle 5 even though it does not directly contact the left ventricle 5.

The second distal electrode pole 202 is implanted in such a position within the septum 13 that it can stimulate the right bundle branch 102 of the heart 1.

If a cardiac stimulation is applied with the electrode 20 in a timeframe in which the heart 1 is susceptible for a ventricular contraction (in particular a contraction of the left ventricle 5), a stimulation pulse applied by the distal bipole 203 will lead to such a ventricular contraction (in particular a contraction of the left ventricle 5). If, however, an electric pulse is applied to the heart 1 in a timeframe in which the heart 1 is not susceptible to a contraction (i.e., in a refractory period), a stimulation applied by the distal bipole 203 will lead to cardiac contractility modulation of the heart 1. This will result in a better contractility of the heart 1 and thus in a better cardiac output of the heart 1 in a subsequent cardiac cycle.

The electrode 20 furthermore comprises a shock coil 210 that is located proximally of the distal bipole 203. This shock coil 210 enables the provision of a defibrillation shock to the heart 1 in case that not only cardiac resynchronization, but also defibrillation is required. Thus, the combined CCM and CRT devise 22 is able to provide a CRT-D therapy. Expressed in other words, the electrode 20 forms part of a combined CCM and CRT-D device 22 as an example of an implantable medical device.

The electrode 20 does not only comprise the first distal electrode pole 201 and the second distal electrode pole 202, but also a first atrial electrode pole 211 and a second atrial electrode pole 212 that both form together an atrial bipole 213. This atrial bipole 213 (which can also be referred to as proximal bipole) serves for detecting atrial signals within the right atrium 2 of the heart 1. By integrating the atrial bipole 213 into the electrode 20, it is not necessary to implant a separate atrial electrode like in case of prior art systems (confer Figure 1). By sensing atrial signals with the atrial bipole 213, the cardiac cycle of the heart 1 can be easily determined. Then, the timing of the different therapies that can be applied by the combined CCM and CRT devise 22 can be adapted to the physiologic needs of the heart 1. Such a timing based on atrial signals facilitates a delivery of stimulation pulses with the distal bipole 203 for providing an LBBAP therapy as well as (during a refractory period of the heart 1) for providing a CCM therapy.

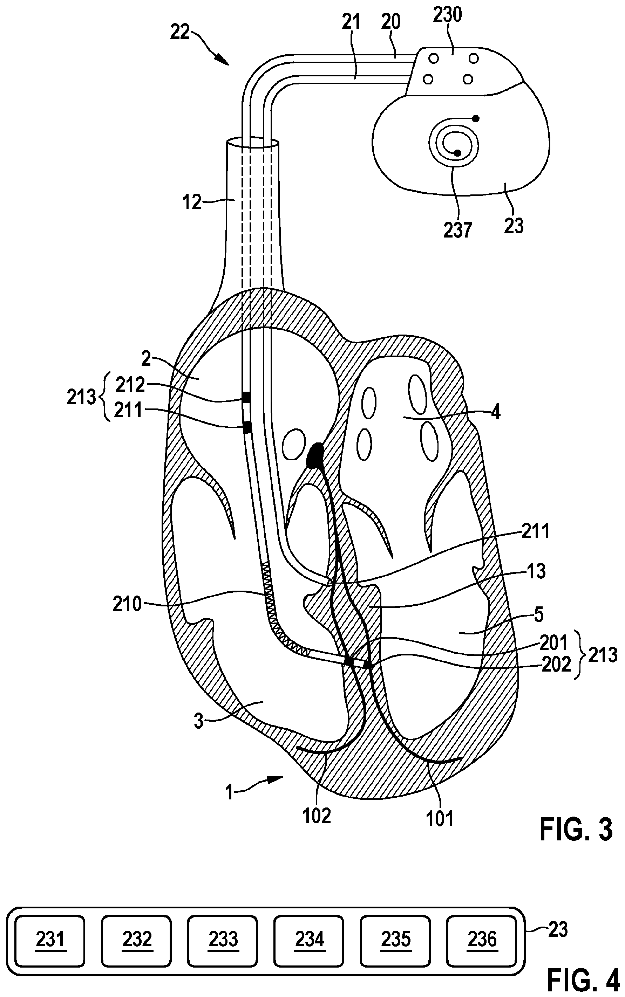

Figure 3 shows another embodiment of a combined CCM and CRT devise 22 that is similar to the embodiment shown in Figure 2. Therefore, only differences between the two embodiments will be explained.

The combined CCM and CRT devise 22 of Figure 3 has a first electrode 20 and a second electrode 21. The first electrode 20 has basically the same configuration as the electrode 20 of the embodiment shown in Figure 2. Thus, the first electrode 20 is particularly appropriate to provide an LBBAP therapy to the heart 1. The second electrode 21 is also guided through the upper vena cava 12 into the heart 1. Here, it is guided through the right atrium 2 and the right ventricle 3 into the septum 13 of the heart 1. The second electrode 21 is secured within the septum 13, however, at a more proximal implantation site than the first electrode 20. The second electrode 21 comprises a distal electrode pole 211 that is configured to emit CCM stimulation pulses. For this purpose, the first distal electrode pole 201 and/or the second distal electrode pole 202 of the distal bipole 203 of the first electrode 20 can be used as counter electrode poles.

Thus, the embodiment shown in Figure 3 has two different electrodes for applying the stimulation pulses for the LBBAP therapy and for the CCM therapy. Nonetheless, the combined CCM and CRT devise 22 features functionalities of cardiac resynchronization via left bundle branch area pacing and cardiac contractility modulation in a particular appropriate manner.

Figure 4 schematically illustrates individual components of an embodiment of an implantable medical device, such as of the embodiments shown in Figures 2 and 3, that are comprised within the stimulator 23 of the implantable medical device 22. The stimulator 23 houses a detection unit 231 (also referred to as sensing unit) that typically comprises an analog-to-digital converter, a bandpass filter, and an offset compensation. The detection unit 231 is operatively connected with a processor 232 that has access to a memory unit 233. The memory unit 233 serves for storing instructions for the processor 232 as well as data detected by the detection unit 231. The stimulation generator 23 further optionally comprises an evaluation unit 234 that can also be part of the processor 232 and that serves for extracting features from the detected cardiac electric signal. The stimulation generator 23 further comprises a stimulation unit 235 that serves for stimulating the heart from which the detection unit 231 detects electric signals. The first electrode 20 and optionally the second electrode 21 (along with their electrode poles 201, 202, and 211; confer Figures 2 and 3) form part of the detection unit 231 and of the stimulation unit 235. Additionally, the stimulation generator 23 comprises a communication unit 236 that serves for data transfer to a (remote) programming device.

## Claims

1. Implantable medical device for stimulating a human or animal heart, comprising a processor (232), a memory unit (233), a stimulation unit (235) configured to stimulate a human or animal heart (1), a detection unit (231) configured to detect an electric signal of the same heart (1), and at least one electrode (20, 21) forming part of the stimulation unit (235) and the detection unit (231),
wherein the at least one electrode (20, 21) comprises a distal bipole (203) and is designed and arranged to be implanted within the septum (13) of the heart (1) to be stimulated and in that the memory unit (233) comprises a computer-readable program that causes the processor (232) to perform the following step when being executed on the processor (232):
a) delivering a left bundle branch area pacing stimulation and a cardiac contractility modulation stimulation to the heart (1) to be stimulated, wherein the cardiac contractility modulation stimulation is delivered temporally offset to the left bundle branch area pacing stimulation.

2. Implantable medical device according to claim 1, **characterized in that** the at least one electrode (20, 21) comprises, besides the distal bipole (203), a proximal bipole (213) being configured to detect an intrinsic atrial signal of the heart (1) to be stimulated.

3. Implantable medical device according to claim 1 or 2, **characterized in that** the at least one electrode (20, 21) comprises, besides the distal bipole (203), a shock coil (210) arranged proximally from the distal bipole (203).

4. Implantable medical device according to any of the preceding claims, **characterized in that** the implantable medical device comprises a second electrode (21) forming part of the stimulation unit (235), wherein the second electrode (21) is designed and arranged to be implanted within the septum (13) of the heart (1) to be stimulated.

5. Implantable medical device according to any of the preceding claims, **characterized in that** the implantable medical device (22) comprises a separate defibrillation electrode forming part of the stimulation unit (235), wherein the separate defibrillation electrode is designed and arranged to be implanted within the right ventricle (3) of the heart (1) to be stimulated.

6. Implantable medical device according to any of claim 1 to 3, **characterized in that** the implantable medical device (22) comprises only a single electrode (20).

7. Implantable medical device according to claim 6, **characterized in that** the implantable medical device (22) comprises a housing (23) with a header (230) having only a single electrode connector receiving socket.

8. Implantable medical device according to any of the preceding claims, **characterized in that** the implantable medical device (22) comprises a rechargeable battery and a device (237) for charging the rechargeable battery by an external charging device.

9. Implantable medical device according to any of the preceding claims, **characterized in that** the computer-readable program causes the processor (232) to detect, with the detection unit (231), a cardiac cycle of the heart (1) to be stimulated; to determine a refractory period of the cardiac cycle; and to deliver the cardiac contractility modulation stimulation within the refractory period of the cardiac cycle.

## Patentansprüche

1. Implantierbares Medizingerät zum Stimulieren eines menschlichen oder tierischen Herzens, umfassend einen Prozessor (232), eine Speichereinheit (233), eine Stimulationseinheit (235), die dazu ausgebildet ist, ein menschliches oder tierisches Herz (1) zu stimulieren, eine Detektionseinheit (231), die dazu ausgebildet ist, ein elektrisches Signal des gleichen Herzens (1) zu detektieren, und mindestens eine Elektrode (20, 21), die einen Teil der Stimulationseinheit (235) und der Detektionseinheit (231) bildet,
wobei die mindestens eine Elektrode (20, 21) einen distalen Bipol (203) umfasst und dazu ausgelegt und angeordnet ist, innerhalb des Septums (13) des zu stimulierenden Herzens (1) implantiert zu werden, und wobei die Speichereinheit (233) ein computerlesbares Programm umfasst, das den Prozessor (232) dazu veranlasst, den folgenden Schritt durchzuführen, wenn es auf dem Prozessor (232) ausgeführt wird:
a) Abgeben einer Schrittmacherstimulation in den Linksschenkelbereich und einer Modulationsstimulation der kardialen Kontraktilität des zu stimulierenden Herzens (1), wobei die Modulationsstimulation der kardialen Kontraktilität zeitlich versetzt zu der Schrittmacherstimulation in den Linksschenkelbereich abgegeben wird.

2. Implantierbares Medizingerät Anspruch 1, **dadurch gekennzeichnet, dass** die mindestens eine Elektrode (20, 21) neben dem distalen Bipol (203) einen proximalen Bipol (213) umfasst, der dazu ausgebildet ist, ein intrinsisches atriales Signal des zu stimulierenden Herzens (1) zu detektieren.

3. Implantierbares Medizingerät nach Anspruch 1 oder 2, **dadurch gekennzeichnet, dass** die mindestens eine Elektrode (20, 21) neben dem distalen Bipol (203) eine Schockspirale (210) umfasst, die proximal von dem distalen Bipol (203) angeordnet ist.

4. Implantierbares Medizingerät nach einem der vorhergehenden Ansprüche, **dadurch gekennzeichnet, dass** dasimplantierbare Medizingerät eine zweite Elektrode (21) umfasst, die einen Teil der Stimulationseinheit (235) bildet, wobei die zweite Elektrode (21) dazu ausgelegt und angeordnet ist, innerhalb des Septums (13) des zu stimulierenden Herzens (1) implantiert zu werden.

5. Implantierbares Medizingerät nach einem der vorhergehenden Ansprüche, **dadurch gekennzeichnet, dass** das implantierbare Medizingerät (22) eine separate Defibrillationselektrode umfasst, die einen Teil der Stimulationseinheit (235) bildet, wobei die separate Defibrillationselektrode dazu ausgelegt und angeordnet ist, innerhalb des rechten Ventrikels (3) des zu stimulierenden Herzens (1) implantiert zu werden.

6. Implantierbares Medizingerät nach einem der Ansprüche 1 bis 3, **dadurch gekennzeichnet, dass** das implantierbare Medizingerät (22) nur eine einzelne Elektrode (20) umfasst.

7. Implantierbares Medizingerät nach Anspruch 6, **dadurch gekennzeichnet, dass** d das implantierbare Medizingerät (22) ein Gehäuse (23) mit einem Header (230) umfasst, welcher nur eine einzelne Buchse aufweist, die den Elektrodenverbinder aufnimmt.

8. Implantierbares Medizingerät nach einem der vorhergehenden Ansprüche, **dadurch gekennzeichnet, dass** das implantierbare Medizingerät (22) eine wiederaufladbare Batterie und eine Vorrichtung (237) zum Laden der wiederaufladbaren Batterie durch eine externe Ladevorrichtung umfasst.

9. Implantierbares Medizingerät nach einem der vorhergehenden Ansprüche, **dadurch gekennzeichnet, dass** das computerlesbare Programm den Prozessor (232) dazu veranlasst, mit der Detektionseinheit (231) einen kardialen Zyklus des zu stimulierenden Herzens (1) zu detektieren; eine Refraktärperiode des kardialen Zyklus zu bestimmen; und die Modulationsstimulation der kardialen Kontraktilität innerhalb der Refraktärperiode des kardialen Zyklus abzugeben.

## Revendications

1. Dispositif médical implantable destiné à stimuler un cœur humain ou animal, comprenant un processeur (232), une unité mémoire (233), une unité de stimulation (235) configurée pour stimuler un cœur humain ou animal (1), une unité de détection (231) configurée pour détecter un signal électrique dudit cœur (1), et au moins une électrode (20, 21) formant une partie de l'unité de stimulation (235) et de l'unité de détection (231),
dans lequel
l'au moins une électrode (20, 21) comprend un bipôle distal (203) et est configurée et agencée pour être implantée au sein du septum (13) du cœur (1) à stimuler et en ce que l'unité mémoire (233) comprend un programme lisible par ordinateur qui amène le processeur (232) à réaliser l'étape suivante lorsqu'il est exécuté sur le processeur (232) :
a) délivrer une stimulation de régulation de zone de branche gauche du faisceau et une stimulation de modulation de contractilité cardiaque au cœur (1) à stimuler, dans lequel la stimulation de modulation de contractilité cardiaque est distribuée de manière décalée dans le temps par rapport à la stimulation de régulation de zone de branche gauche du faisceau.

2. Dispositif médical implantable selon la revendication 1, **caractérisé en ce que** l'au moins une électrode (20, 21) comprend, à côté du bipôle distal (203), un bipôle proximal (213) configuré pour détecter un signal auriculaire intrinsèque du cœur (1) à stimuler.

3. Dispositif médical implantable selon la revendication 1 ou 2, **caractérisé en ce que** l'au moins une électrode (20, 21) comprend, à côté du bipôle distal (203), une bobine de choc (210) agencée de manière proximale par rapport au bipôle distal (203).

4. Dispositif médical implantable selon l'une quelconque des revendications précédentes, **caractérisé en ce que** le dispositif médical implantable comprend une seconde électrode (21) formant une partie de l'unité de stimulation (235), dans lequel la seconde électrode (21) est conçue et agencée pour être implantée au sein du septum (13) du cœur (1) à stimuler.

5. Dispositif médical implantable selon l'une quelconque des revendications précédentes, **caractérisé en ce que** le dispositif médical implantable (22) comprend une électrode de défibrillation distincte formant une partie de l'unité de stimulation (235), dans lequel l'électrode de défibrillation distincte est conçue et agencée pour être implantée au sein du ventricule droit (3) du cœur (1) à stimuler.

6. Dispositif médical implantable selon l'une quelconque des revendications 1 à 3, **caractérisé en ce que** le dispositif médical implantable (22) comprend uniquement une électrode unique (20).

7. Dispositif médical implantable selon la revendication 6, **caractérisé en ce que** le dispositif médical implantable (22) comprend un boîtier (23) avec un collecteur (230) ayant uniquement une douille de réception de connecteur d'électrode unique.

8. Dispositif médical implantable selon l'une quelconque des revendications précédentes, **caractérisé en ce que** le dispositif médical implantable (22) comprend une batterie rechargeable et un dispositif (237) destiné à charger la batterie rechargeable par le biais d'un dispositif de charge externe.

9. Dispositif médical implantable selon l'une quelconque des revendications précédentes, **caractérisé en ce que** le programme lisible par ordinateur amène le processeur (232) à détecter, avec l'unité de détection (231), un cycle cardiaque du cœur (1) à stimuler ; à déterminer une phase réfractaire du cycle cardiaque ; et à distribuer la stimulation de modulation de contractilité cardiaque au sein de la phase réfractaire du cycle cardiaque.
